# EUROPEAN PATENT APPLICATION

(11) **EP 2 031 728 A2**
(43) Date of publication of application: **04.03.2009**
(21) Application number: 08015354.7
(22) Date of filing: 29.08.2008
(51) Int. Cl.: H02J 5/00, H02J 7/00

(54) **Wirelessly chargeable and portable ultrasonic diagnostic device**

(30) Priority: 31.08.2007 KR 20070088325
(71) Applicant: MEDISON CO., LTD., Kangwon-do 250-870 (KR)
(72) Inventor: Shin, Soo, Hwan, Gangnam-gu Seoul 135-280 (KR); Song, Young, Seuk, Gangnam-gu Seoul 135-280 (KR); Lee, Jin, Yong, Gangnam-gu Seoul 135-280 (KR)
(74) Representative: Lorenz, Markus

(57) **Abstract**

Embodiments of the present invention may provide a portable ultrasonic diagnostic device to which electric energy can be transferred wirelessly. The device according to the present invention comprises: a wireless energy radiating part for converting an electrical energy into wireless energy to radiate the wireless energy therearound; a main body of the portable ultrasonic diagnostic device, the main body being separated from the radiating part; a wireless energy converting part disposed in the main body and being configured to convert the wireless energy radiated from the radiating part into the electrical energy; and a rechargeable battery connected to the converting part so as to be recharged by the electrical energy converted at the converting part.

## Description

The present application claims priority from Korean Patent Application No. 10-2007-0088325 filed on August 31, 2007, the entire subject matter of which is incorporated herein by reference.

### BACKGROUND

### 1. Field

The present invention generally relates to a portable ultrasonic diagnostic device, and more particularly to a portable ultrasonic diagnostic device which is capable of being charged wirelessly.

### 2. Background

An ultrasonic diagnostic device is used for obtaining an ultrasonic image of a target region in order to provide clinical information of said region (e.g., lesion or neoplasm information of internal organs, fetus information, etc.). Typically, the ultrasonic diagnostic device comprises at least one probe for radiating ultrasonic waves to the target region and receiving an echo signal reflected from the target region. The probe has a transducer for converting an ultrasonic signal into an electric signal. Further, certain techniques for acquiring a three-dimensional (3D) ultrasonic image by pivoting the transducer have been developed to obtain more accurate diagnosis.

Recently, there have been introduced portable ultrasonic diagnostic devices, which have configurations similar to laptop computers. During use, the portable ultrasonic diagnostic device is typically placed on a support station. When necessary, the portable ultrasonic diagnostic device can be separated from the support station and be moved to another location.

The portable ultrasonic diagnostic device may use batteries, which are rechargeable with electrical energy.

The electrical energy is used for operating the portable ultrasonic diagnostic device and radiating ultrasonic waves from the ultrasonic probe. The electrical energy is also used for pivoting the transducer of the ultrasonic probe in order to obtain three-dimensional images.

However, although a significant amount of electrical energy is required in the portable ultrasonic diagnostic device, the rechargeable battery has a limited capacity. Sometimes, this causes diagnosis to be stopped so that the battery can be recharged.

### BRIEF DESCRIPTION OF THE DRAWINGS

Arrangements and embodiments may be described in detail with reference to the following drawings in which like reference numerals refer to like elements and wherein:

Fig. 1 is a block diagram of a portable ultrasonic diagnostic device in accordance with a first embodiment of the present invention.

Fig. 2 is a side view of the portable ultrasonic diagnostic device shown in Fig. 1.

Fig. 3 is a block diagram of a portable ultrasonic diagnostic device in accordance with a second embodiment of the present invention.

Fig. 4 is a perspective view of the portable ultrasonic diagnostic device shown in Fig. 3.

### DETAILED DESCRIPTION OF THE PRESENT INVENTION

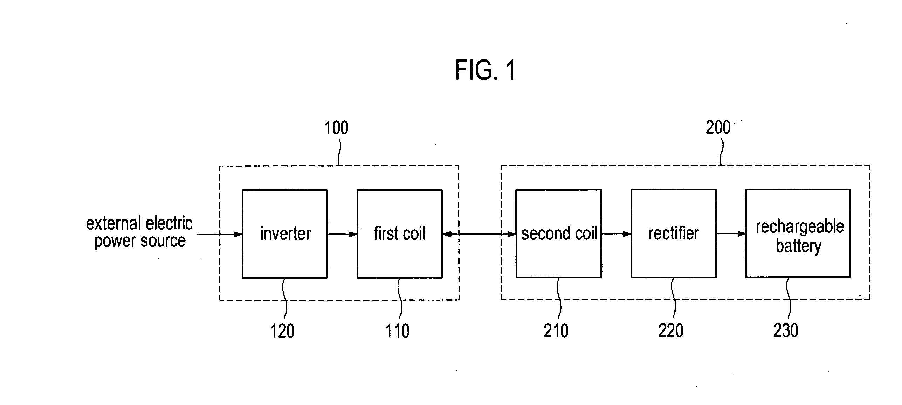

A wirelessly chargeable portable ultrasonic diagnostic device, which is constructed in accordance with the present invention, will now be described in detail with reference to Figs. 1 to 4.

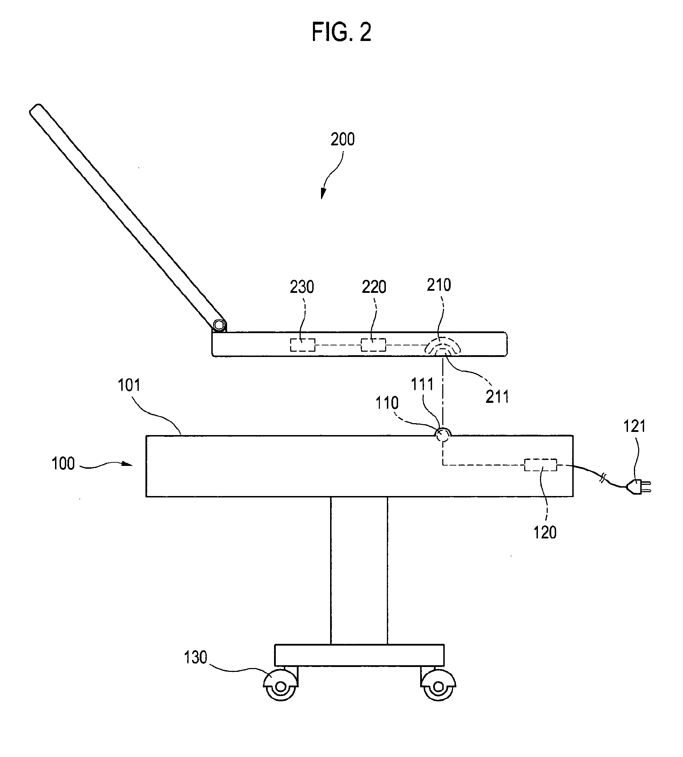

Fig. 1 is a block diagram of a portable ultrasonic diagnostic device in accordance with a first embodiment of the present invention. Fig. 2 is a side view of the portable ultrasonic diagnostic device shown in Fig. 1.

As shown in Figs. 1 and 2, the portable ultrasonic diagnostic device in accordance with the first embodiment can be recharged by induced electricity. The device includes a main body 200 and a support station 100. The main body 200 of the device can be placed on the support station 100 for recharge. The support station 100 includes a first coil 110 and an inverter 120 which receives A. C. voltage from an external electric power source by a cable 121 and supplies the A. C. voltage to the first coil with a predetermined amplitude. Thus, when the A. C. voltage from the inverter 120 is applied to the first coil 110, the first coil 110 generates magnetic fields therearound. The main body 200 includes a second coil 210 and a rectifier 220 connected to the second coil 210.

When the main body 200 is placed on the support station 100, the second coil faces the first coil 110. Thus, when the first coil generates magnetic fields, the second coil 210 generates induced electricity (which is A. C voltage) in the magnetic fields. Then, the rectifier 220 converts the induced electricity of the second coil 210 into D. C. voltage.

The rectifier 220 is connected to a rechargeable battery 230 (e.g., a capacitor). Thus, the rechargeable battery 230 can be recharged with D. C voltage converted by the rectifier 220.

The support station 100 includes a charging coupler 111 which is protruded at a position corresponding to the first coil 110. The main body 200 includes a charging port 211 which is recessed at a position corresponding to the second coil 210. The charging coupler 111 can be inserted into the charging port 211. Thus, when the main body 200 is placed on the support station 100 with insertion of the charging coupler 111 into the charging port 211, the first coil 110 precisely faces the second coil 210. Alternatively, the main body may include a charging coupler and the support station may include a charging port.

When the external electric power source supplies A. C. voltage to the inverter 120, the inverter 120 may supply A. C. voltage to the first coil 110. Then, variable electric fields may be generated at the first coil 110 by the A. C. voltage. The variable electric fields at the first coil 110 may generate variable magnetic fields around the first coil 110. Then, the energy of the variable magnetic fields may induce A.C. voltage at the second coil 210 adjacent to the first coil 110. The rectifier 220 may convert the induced A. C. voltage of the second coil 210 into D. C. voltage. The rechargeable battery 230 may be recharged by the D. C. voltage from the rectifier 220. As such, the rechargeable battery 230 can be recharged wirelessly.

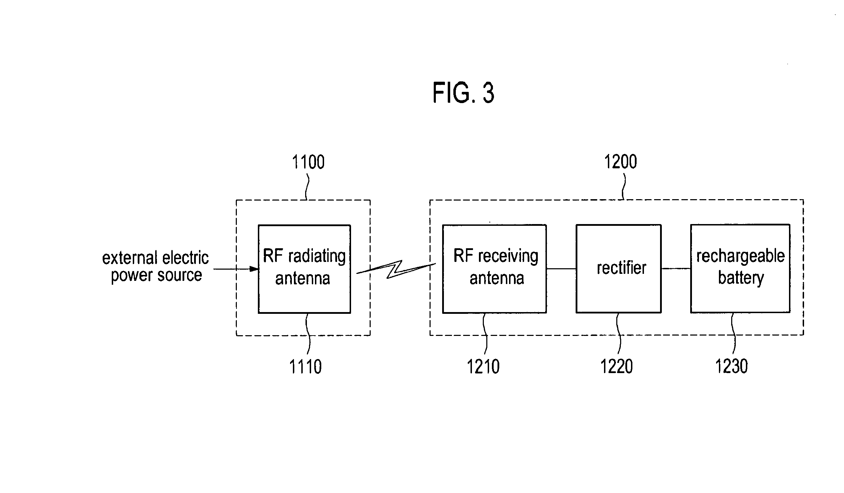

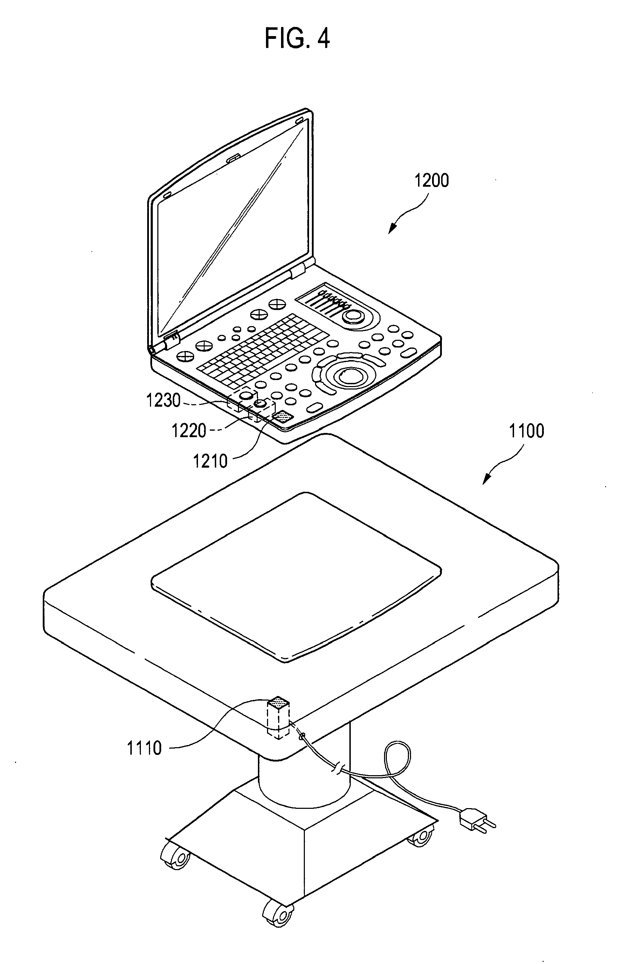

Fig. 3 is a block diagram of a portable ultrasonic diagnostic device in accordance with a second embodiment of the present invention. Fig. 4 is a perspective view of the portable ultrasonic diagnostic device shown in Fig. 3.

As shown in Figs. 3 and 4, the portable ultrasonic diagnostic device in accordance with the second embodiment can be recharged by RF (radio-frequency) tuning. The device includes a main body 1200 and a support station 1100. The main body 1200 of the device can be placed on the support station 1100. The support station 1100 includes an electromagnetic wave radiating part 1110 (e.g., RF radiating antenna) which converts electric energy from an external electric power source into electromagnetic waves and radiates the waves therearound in a constant frequency. For converting the energy of the radiated electromagnetic waves into electric voltage, the main body 1200 includes an electromagnetic wave receiving part 1210 (e.g., RF receiving antenna) which generates tuned signals by resonance in a frequency corresponding to the frequency of the radiated electromagnetic waves from the radiating part 1110. A rectifier 1220 is connected to the receiving part 1210 for smoothing the tuned signals of the receiving part 1210 and converting the signals into D. C voltage. A rechargeable battery 1230 is connected to the rectifier 1220 and may be recharged with the D. C. voltage converted by the rectifier 1220. The rechargeable battery 1230 supplies the electric power to operate the portable ultrasonic diagnostic device.

When the main body 1200 is placed on or adjacent to the support station 1100, the receiving part 1210 can be tuned by resonance with the electromagnetic wave since the radiating part 1110 radiates electromagnetic waves. Then, the rectifier 1220 converts the tuned signals of the receiving part into D. C. voltage. Thus, the rechargeable battery 1230 can be recharged wirelessly with the D. C. voltage of rectifires 1220. As such, a diagnosis need not be stopped for recharging the rechargeable battery 1230.

Embodiments of the present invention may provide a wirelessly chargeable portable ultrasonic diagnostic device. The device comprises: a wireless energy radiating part which converts electric energy into wireless energy and radiates the wireless energy therearound; a main body of the portable ultrasonic diagnostic device, the main body being separated from the radiating part; a wireless energy converting part disposed in the main body which converts the wireless energy radiated from the radiating part into an electric energy; and a rechargeable battery connected to the converting part for being recharged by the electric energy converted at the converting part.

The wireless energy radiating part may include a first coil receiving A.C. voltage from an external electric power source for generating magnetic fields therearound. The converting part may include a second coil for inducing A. C. voltage in the magnetic fields around the first coil and a rectifier for converting the induced A. C. voltage of the second coil into D.C voltage to supply the D. C. voltage to the rechargeable battery. The rechargeable battery may be recharged by the D.C voltage from the rectifier.

The radiating part has a charging coupler which is protruded at a position corresponding to the first coil. The converting part has a charging port which is recessed at a position corresponding to the second coil. Oppositely, the converting part has a charging coupler which is protruded at a position corresponding to the second coil, while the radiating part has a charging port which is recessed at a position corresponding to the first coil. The charging couplers can be inserted into the charging ports.

Alternatively, the wireless energy radiating part radiates electromagnetic waves in a constant frequency. The converting part includes a receiving part for generating tuned signals by resonance in a frequency corresponding to the frequency of radiated electromagnetic waves. It also includes a rectifier for smoothing the tuned signals and converting the signals into D. C. voltage to supply the D. C. voltage to the rechargeable battery. The rechargeable battery can be recharged by the D.C voltage from the rectifier.

The wireless energy radiating part can be mounted to a support station where the main body may be placed.

Although embodiments of the present invention have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A portable ultrasonic diagnostic device which is chargeable wirelessly.

2. A wirelessly chargeable portable ultrasonic diagnostic device, comprising:
a wireless energy radiating part for converting an electrical energy into wireless energy to radiate the wireless energy therearound;
a main body of the portable ultrasonic diagnostic device, the main body being separated from the radiating part;
a wireless energy converting part disposed in the main body and configured to convert the wireless energy radiated from the radiating part into the electrical energy; and
a rechargeable battery connected to the converting part for being recharged by the electrical energy converted at the converting part.

3. The device of Claim 2, wherein the wireless energy radiating part includes a first coil receiving A.C. voltage from an external electric power source for generating magnetic fields therearound;
wherein the converting part includes a second coil for inducing A. C. voltage in the magnetic fields around the first coil and a rectifier which converts the induced A. C. voltage of the second coil into D.C voltage to supply the D. C. voltage to the rechargeable battery; and
wherein the rechargeable battery is recharged by the D.C voltage from the rectifier.

4. The device of Claim 3, wherein the radiating part has a charging coupler protruded at a position corresponding to the first coil while the converting part has a charging port recessed at a position corresponding to the second coil, the charging coupler being inserted into the charging port.

5. The device of Claim 3, wherein the converting part has a charging coupler protruded at a position corresponding to the second coil while the radiating part has a charging port recessed at a position corresponding to the first coil, the charging coupler being inserted into the charging port.

6. The device of Claim 2, wherein the wireless energy radiating part radiates electromagnetic waves in a constant frequency;
wherein the converting part includes a receiving part for generating tuned signals by resonance in a frequency corresponding to the frequency of radiated electromagnetic waves and a rectifier for smoothing the tuned signals and converting the signals into D. C. voltage to supply the D. C. voltage to the rechargeable battery;
and wherein the rechargeable battery is recharged by the D.C voltage from the rectifier.

7. The device of any one of Claims 2 to 6, wherein the wireless energy radiating part is mounted to a support station where the main body is placed.
